# EUROPEAN PATENT APPLICATION

(11) **EP 2 363 489 A1**
(43) Date of publication of application: **07.09.2011**
(21) Application number: 10155377.4
(22) Date of filing: 03.03.2010
(51) Int. Cl.: C12N 15/54, C12N 9/12, C12P 13/08, C12N 1/21

(54) **Enzyme Aspartokinase III having a reduced L-lysine feedback inhibition**

(71) Applicant: Technische Universität Hamburg-Harburg, 21073 Hamburg (DE); TuTech Innovation GmbH, 21079 Hamburg (DE)
(72) Inventor: Zeng, An-Ping, 21071 Hamburg (DE); Chen, Zhen, 21071 Hamburg (DE)
(74) Representative: UEXKÜLL & STOLBERG

(57) **Abstract**

The present invention relates to methods and enzymes which are useful for producing L-lysine and L-threonine. In particular, the present invention provides a modified aspartokinase III enzyme with a reduced sensitivity to L-lysine feedback inhibition. The invention also provides methods for producing L-lysine and L-threonine which make use of the modified aspartokinase III enzyme. The invention also relates to the use of the modified aspartokinase III enzyme for producing L-lysine and L-threonine.

SEQUENCE LISTING

<110>  Technische Universität Hamburg-Harburg
       TuTech Innovation GmbH

<120>  Aspartokinase III with a reduced feedback inhibition sensitivity

<130>  P 81349

<160>  21

<170>  PatentIn version 3.3

<210>  1
<211>  449
<212>  PRT
<213>  Escherichia coli

<400>  1

Met Ser Glu Ile Val Val Ser Lys Phe Gly Gly Thr Ser Val Ala Asp
1               5                   10                  15

Phe Asp Ala Met Asn Arg Ser Ala Asp Ile Val Leu Ser Asp Ala Asn
            20                  25                  30

Val Arg Leu Val Val Leu Ser Ala Ser Ala Gly Ile Thr Asn Leu Leu
        35                  40                  45

Val Ala Leu Ala Glu Gly Leu Glu Pro Gly Glu Arg Phe Glu Lys Leu
    50                  55                  60

Asp Ala Ile Arg Asn Ile Gln Phe Ala Ile Leu Glu Arg Leu Arg Tyr
65                  70                  75                  80

Pro Asn Val Ile Arg Glu Glu Ile Glu Arg Leu Leu Glu Asn Ile Thr
                85                  90                  95

Val Leu Ala Glu Ala Ala Ala Leu Ala Thr Ser Pro Ala Leu Thr Asp
            100                 105                 110

Glu Leu Val Ser His Gly Glu Leu Met Ser Thr Leu Leu Phe Val Glu
        115                 120                 125

Ile Leu Arg Glu Arg Asp Val Gln Ala Gln Trp Phe Asp Val Arg Lys
    130                 135                 140

Val Met Arg Thr Asn Asp Arg Phe Gly Arg Ala Glu Pro Asp Ile Ala
145                 150                 155                 160

Ala Leu Ala Glu Leu Ala Ala Leu Gln Leu Leu Pro Arg Leu Asn Glu
                165                 170                 175

Gly Leu Val Ile Thr Gln Gly Phe Ile Gly Ser Glu Asn Lys Gly Arg
            180                 185                 190

Thr Thr Thr Leu Gly Arg Gly Gly Ser Asp Tyr Thr Ala Ala Leu Leu
        195                 200                 205

Ala Glu Ala Leu His Ala Ser Arg Val Asp Ile Trp Thr Asp Val Pro
    210                 215                 220

Gly Ile Tyr Thr Thr Asp Pro Arg Val Val Ser Ala Ala Lys Arg Ile
225                 230                 235                 240

Asp Glu Ile Ala Phe Ala Glu Ala Ala Glu Met Ala Thr Phe Gly Ala
                245                 250                 255

Lys Val Leu His Pro Ala Thr Leu Leu Pro Ala Val Arg Ser Asp Ile
            260                 265                 270

Pro Val Phe Val Gly Ser Ser Lys Asp Pro Arg Ala Gly Gly Thr Leu
        275                 280                 285

Val Cys Asn Lys Thr Glu Asn Pro Pro Leu Phe Arg Ala Leu Ala Leu
    290                 295                 300

Arg Arg Asn Gln Thr Leu Leu Thr Leu His Ser Leu Asn Met Leu His
305                 310                 315                 320

Ser Arg Gly Phe Leu Ala Glu Val Phe Gly Ile Leu Ala Arg His Asn
                325                 330                 335

Ile Ser Val Asp Leu Ile Thr Thr Ser Glu Val Ser Val Ala Leu Thr
            340                 345                 350

Leu Asp Thr Thr Gly Ser Thr Ser Thr Gly Asp Thr Leu Leu Thr Gln
        355                 360                 365

Ser Leu Leu Met Glu Leu Ser Ala Leu Cys Arg Val Glu Val Glu Glu
    370                 375                 380

Gly Leu Ala Leu Val Ala Leu Ile Gly Asn Asp Leu Ser Lys Ala Cys
385                 390                 395                 400

Gly Val Gly Lys Glu Val Phe Gly Val Leu Glu Pro Phe Asn Ile Arg
                405                 410                 415

Met Ile Cys Tyr Gly Ala Ser Ser His Asn Leu Cys Phe Leu Val Pro
            420                 425                 430

Gly Glu Asp Ala Glu Gln Val Val Gln Lys Leu His Ser Asn Leu Phe
        435                 440                 445

Glu


<210>  2
<211>  32
<212>  DNA

<213>  Artificial

<220>
<223>  oligonucleotide primer

<400>  2
gagcctcata tgtctgaaat tgttgtctcc aa                                    32


<210>  3
<211>  32
<212>  DNA
<213>  Artificial

<220>
<223>  oligonucleotide primer

<400>  3
catgctctcg agctcaaaca aattactatg ca                                    32


<210>  4
<211>  36
<212>  DNA
<213>  Artificial

<220>
<223>  oligonucleotide primer

<400>  4
acagcctgaa tatgctggct tctcgcggtt tcctcg                                36


<210>  5
<211>  36
<212>  DNA
<213>  Artificial

<220>
<223>  oligonucleotide primer

<400>  5
cgaggaaacc gcgagaagcc agcatattca ggctgt                                36


<210>  6
<211>  38
<212>  DNA
<213>  Artificial

<220>
<223>  oligonucleotide primer

<400>  6
catcctcgcg cggcataata ttttggtaga cttaatca                              38

<210>  7
<211>  38
<212>  DNA
<213>  Artificial

<220>
<223>  oligonucleotide primer

<400>  7
tgattaagtc taccaaaata ttatgccgcg cgaggatg                                38


<210>  8
<211>  37
<212>  DNA
<213>  Artificial

<220>
<223>  oligonucleotide primer

<400>  8
cgcggcataa tatttcggca gacttaatca ccacgtc                                 37


<210>  9
<211>  37
<212>  DNA
<213>  Artificial

<220>
<223>  oligonucleotide primer

<400>  9
gacgtggtga ttaagtctgc cgaaatatta tgccgcg                                 37


<210>  10
<211>  40
<212>  DNA
<213>  Artificial

<220>
<223>  oligonucleotide primer

<400>  10
cgaagcggca gagatggcac gttttggtgc aaaagtactg                              40


<210>  11
<211>  40
<212>  DNA
<213>  Artificial

<220>
<223>  oligonucleotide primer

<400>  11
cagtactttt gcaccaaaac gtgccatctc tgccgcttcg                              40


<210>  12
<211>  28
<212>  DNA
<213>  Artificial

<220>
<223>  oligonucleotide primer

<400>  12
ccgcgctctg gcgcttgctc gcaatcag                                           28


<210>  13
<211>  28
<212>  DNA
<213>  Artificial

<220>
<223>  oligonucleotide primer

<400>  13
ctgattgcga gcaagcgcca gagcgcgg                                           28


<210>  14
<211>  33
<212>  DNA
<213>  Artificial

<220>
<223>  oligonucleotide primer

<400>  14
cgcggtttcc tcgcggaagt cgtcggcatc ctc                                     33


<210>  15
<211>  33
<212>  DNA
<213>  Artificial

<220>
<223>  oligonucleotide primer

<400>  15
gaggatgccg acgacttccg cgaggaaacc gcg                                     33


<210>  16
<211>  39
<212>  DNA

<213>  Artificial

<220>
<223>  oligonucleotide primer

<400>  16
ggcatcctcg cgcggcataa tccttcggta gacttaatc                              39


<210>  17
<211>  39
<212>  DNA
<213>  Artificial

<220>
<223>  oligonucleotide primer

<400>  17
gattaagtct accgaaggat tatgccgcgc gaggatgcc                              39


<210>  18
<211>  39
<212>  DNA
<213>  Artificial

<220>
<223>  oligonucleotide primer

<400>  18
ggcatcctcg cgcggcataa tccttcggta gacttaatc                              39


<210>  19
<211>  39
<212>  DNA
<213>  Artificial

<220>
<223>  oligonucleotide primer

<400>  19
gattaagtct accgaaggat tatgccgcgc gaggatgcc                              39


<210>  20
<211>  41
<212>  DNA
<213>  Artificial

<220>
<223>  oligonucleotide primer

<400>  20
ggcgaattca aaggaggaaa atcatgtctg aaattgttgt c                           41

<210>  21
<211>  32
<212>  DNA
<213>  Artificial

<220>
<223>  oligonucleotide primer

<400>  21
ggcggatcct tactcaaaca aattactatg ca                                    32

## Description

### TECHNICAL FIELD

The present invention relates to methods and enzymes which are useful for producing L-lysine and L-threonine. In particular, the present invention provides a modified aspartokinase III enzyme with a reduced sensitivity to L-lysine feedback inhibition. The invention also provides methods for producing L-lysine and L-threonine which make use of the modified aspartokinase III enzyme. The invention also relates to the use of the modified aspartokinase III enzyme for producing L-lysine and L-threonine.

### BACKGROUND OF THE INVENTION

Amino acids are the basic building blocks for proteins. These nutritional key compounds are widely used for the preparation of feed additives, food ingredients, nutraceuticals and pharmaceuticals. The world production of amino acids has been steadily increasing in recent years and has reached approximately 2 million tons per year. In particular, the amino acids of the aspartic acid group (including L-lysine and L-threonine) have found wide applications, and several hundred thousand tons of these amino acids are produced per year worldwide by microbial fermentation using organisms such as *Corynebacterium glutamicum* and *Escherichia coli.*

The biosynthesis of amino acids of the aspartic acid group in *E. coli* follows a cascade of biochemical reactions involving the conversion of aspartic acid to β-phosphoaspartic acid. This reaction, which is regarded as a key regulatory step in the biosynthetic pathway, is catalyzed by the enzyme aspartokinase. There are three aspartokinase isoforms in *E*. *coli* (referred to as aspartokinases I, II, III): aspartokinases I and II are bifunctional enzymes which also exhibit a homoserine dehydrogenase activity. Aspartokinase I is encoded by the *thrA* gene and is inhibited by threonine, and its synthesis is repressed by a combined action of threonine and isoleucine (F. Falcoz-Kelly et al. (1969), Eur. J. Biochem. 8:146-152). Aspartokinase II is encoded by the *metL* gene, and its synthesis is repressed by methionine (J. C. Patte et al. (1967), Biochim. Biophys. Acta., 136:245-257). Aspartokinase III is encoded by the *lysC* gene, and it is both repressed and inhibited by L-lysine (Truffa-Bachi et al. (1968), Eur. J. Biochem. 5:73-80).

Today, the release of aspartokinase III from feedback control mechanisms is regarded as one of the most important features of industrial lysine producer strains. This is also underlined by various patents and patent applications relating to promoter modifications or amino acid exchanges in the primary structure of the relevant biosynthetic enzymes. For example, US patent 5,661,012 discloses that an amino acid replacement at certain positions of the aspartokinase III of *E*. *coli* releases the activity of the enzyme from feedback inhibition by L-lysine. US patent 6,040,160 discloses microorganisms which harbour both a mutated aspartokinase III and a mutated dihydrodipicolinate synthase, wherein the mutations in these enzymes desensitize the feedback inhibition by L-lysine.

Despite these improvements, novel means and methods would be desirable which provide for a release of the biosynthesis process of producing L-lysine and L-threonine from feedback inhibition by L-lysine. The present invention provides novel aspartokinase III enzymes which have been obtained by modifying the amino acid sequence of the aspartokinase III of *E*. *coli* by amino acid substitutions other than those described in the art. Thus, the present invention provides further alternatives for optimizing the production process of L-lysine and L-threonine. By introducing genes coding for these modified enzymes into L-lysine and/or L-threonine producing bacteria, the fermentative production of these amino acids can be significantly improved.

### DETAILED DESCRIPTION OF THE INVENTION

The present invention provides improved aspartokinase III enzymes which have been derived from the *E*. *coli* enzyme depicted in SEQ ID NO:1 by one or more amino acid substitutions at predetermined amino acid positions. Specifically, it was found that an amino acid substitution in any of amino acid positions 253, 305, 320, 329, 337, 338, 339 and 346 of the aspartokinase III enzyme from *E*. *coli* results in a modified enzyme which has a significantly reduced L-lysine feedback inhibition sensitivity. This means that compared to the wild-type enzyme, higher concentrations of L-lysine are required to inhibit the enzymatical activity of the enzyme. Some of the mutated enzymes were even found to be completely released from L-lysine feedback inhibition. Releasing the aspartokinase III enzyme from L-lysine feedback inhibition, either partially or completely, is a particularly suitable way of increasing the yield of the amino acids L-lysine and L-threonine in a microorganism-based production process, as aspartokinase III catalyses a decisive step in the biosynthesis of both amino acids.

The amino acid sequence shown herein as SEQ ID NO:1 is the amino acid sequence of wild-type aspartokinase III of Es*cherichia coli.* This enzyme has been widely used in the art for the production of the amino acid L-lysine and L-threonine. In order to enhance the product yield during the production of L-lysine and L-threonine, several amino acid substitutions in the sequence of SEQ ID NO:1 have been suggested in the art for releasing the enzyme from the feedback inhibition by L-lysine. For example, US patent 5,661,012 discloses amino acid substitutions at positions 318, 323, 325, 345, 347 and 349 of SEQ ID NO:1. The present invention has identified additional positions which can be mutated to achieve a significant or complete release from feedback inhibition effected by L-lysine. Therefore, the present invention provides novel enzymatically active polypeptides and enzymatically active fragments thereof which are useful for providing an enhanced production of L-lysine and L-threonine. Based on the present disclosure, genetically modified microorganisms, in particular strains of *E*. *coli or C. glutamicum,* can be generated for use in optimized fermentative production processes.

Host cells which express the aspartokinase III enzyme modified according to the invention will produce both L-lysine and L-threonine upon culturing of the cells in a suitable production medium. While L-lysine is normally obtained in high yields, the amount of L-threonine recovered from the culture will be lower. Consequently, additional modifications may be required to increase the yield for L-threonine. For example, it may be required to provide the producing cells with one or more additional copies of the phosphoenolpyruvate carboxylase gene and the threonine operon, as described in US 7,011,961. In addition, it may also be required to provide for a mutated aspartokinase I enzyme which has a reduced L-threonine feedback inhibition sensitivity, as described in US 5,175,107, US 7,186,531, US 7,220,571.

In a first aspect, the invention thus provides a polypeptide which is an enzymatically active aspartokinase III with a reduced sensitivity to L-lysine feedback inhibition, said polypeptide comprising the amino acid sequence of SEQ ID NO:1 or an enzymatically active fragment or homolog thereof, wherein one or more amino acids have been replaced at positions that correspond to amino acids 253, 305, 320, 329, 337, 338, 339 and 346 of SEQ ID NO:1. In general, an amino acid occurring at one of the positions which correspond to positions 253, 305, 320, 329, 337, 338, 339 or 346 of SEQ ID NO:1 can be replaced by any other naturally or non-naturally occurring amino acids or by an amino acid analogue, provided that the modified aspartokinase resulting from the replacement is still enzymatically active and exhibits a reduced sensitivity with respect to the feedback inhibition imposed by L-lysine.

As the modified aspartokinase enzymes provided by the present invention are for use in production processes of L-lysine and L-threonine, it is crucial that the above-mentioned amino acid substitutions, either at positions relevant for L-lysine feedback inhibition or not, do not result in a significant loss of activity of the enzyme due to a spatial rearrangement of the polypeptide structure. The polypeptides provided by the invention are enzymatically active, which means that they have retained at least part of the enzymatic activity of the aspartokinase III depicted in SEQ ID NO:1 (as measured in the absence of L-lysine). Preferably, the polypeptides provided by the present invention have retained 50%, 60%, 70%, 80%, 90%, 95%, 96%, 97%, 98%, or 99% of the activity of the aspartokinase III of SEQ ID NO:1, as measured in the absence of L-lysine. According to a particularly preferred embodiment, the amino acid replacements disclosed by the present invention, which result in a complete or partial release from L-lysine feedback inhibition, have no influence on the enzymatic activity of the mutated aspartokinase.

It has been found that the aspartokinase enzymes of the invention, which have been modified by the replacement of any of the amino acids at positions 253, 305, 320, 329, 337, 338, 339 and 346 of SEQ ID NO:1, show a significant reduction in sensitivity to L-lysine feedback inhibition. Preferably, the polypeptide enzymes of the invention are at least 2-fold, at least 5-fold, at least 10-fold, at least 15-fold, at least 20-fold, at least 25-fold, at least 50-fold, at least 75-fold, at least 100-fold, at least 150-fold, at least 200-fold, at least 250-fold, at least 500-fold, at least 750-fold, at least 1000-fold, or at least 5000-fold less sensitive to L-lysine feedback inhibition as compared to the original enzyme shown in SEQ ID NO:1 when measured, for example, in the assay described in the examples below. Even more preferably, the polypeptides of the invention are substantially free from L-lysine feedback inhibition (i.e. resistant), which means that the presence of lysine in the culture medium does not impair or otherwise negatively influence the enzyme activity of the mutated aspartokinase enzymes.

The polypeptide of the invention may comprise an amino acid sequence as shown in SEQ ID NO:1, in which one or more of the amino acids located in positions 253, 305, 320, 329, 337, 338, 339 and 346 of SEQ ID NO:1 have been replaced by other amino acids. The amino acids which can be used for replacing the respective amino acids in the naturally occurring enzyme of *E*. *coli* are not limited to specific amino acids. In principle, any other proteinogenic or non-proteinogenic amino acid may be used for substituting the naturally occurring amino acid in the respective position of the enzyme. Preferably, the amino acids found in the original enzyme of *E*. *coli* can be replaced by any other naturally occurring, proteinogenic amino acid. As used herein, proteinogenic amino acids are those 23 amino acids which are regularly found in naturally occurring polypeptides, i.e. isoleucine (Ile), alanine (Ala), leucine (Leu), asparagine (Asn), lysine (Lys), aspartic acid (Asp), methionine (Met), cysteine (Cys), phenylalanine (Phe), glutamic acid (Glu), threonine (Thr), glutamine (Gln), tryptophan (Trp), glycine (Gly), valine (Val), proline (Pro), serine (Ser), tyrosine (Tyr), arginine (Arg), histidine (His), selenocysteine, selenomethionine, and pyrrollysine. Preferably, the amino acids are L-amino acids. However, also D-amino acids may be useful for replacing the amino acids in the original polypeptide of SEQ ID NO:1.

Alternatively, the amino acids used for replacing the amino acids in the naturally occurring enzyme of *E*. *coli* are non-proteinogenic amino acids, i.e. amino acids which are not found in naturally occurring polypeptides. These non-proteinogenic amino acids include, for example, α-aminoadipic acid, β-aminoadipic acid, α-aminobutyric acid, α-aminoisobutyric acid, β-alanine, 4-aminobutyric acid, 5-aminovaleric acid, 6-aminohexanoic acid, 8-aminooctanoic acid, 9-aminononanoic acid, 10-aminodecanoic acid, 12-aminododecanoic acid, α-aminosuberic acid, β-cyclohexylalanine, citrulline, dehydroalanine, α-cyclohexylglycine, propargylglycine, pyroglutamic acid, 4-benzoylphenylalanine, δ-hydroxylysine, 4-hydroxyproline, alloisoleucine, lanthionine (Lan), norleucine, norvaline, or-nithine, phenylglycin, pipecolic acid, sarcosine, 1,2,3,4-tetrahydro-isochinoline-3-carboxylic acid, allo-threonine, thiazolidine-4-carboxylic acid, γ-aminobutyric acid (GABA), iso-cysteine, diaminopropionic acid, 2,4-diaminobutyric acid, 3,4-diaminobutyric acid, biphenylalanine and 4-fluoro-phenylalanine.

Also included by the term "non-proteinogenic amino acids" are derivatives of the above-mentioned proteinogenic amino acids wherein a side-chain has been modified, for example, by a methylene group, thereby providing e.g. homomethionine, homoserine, homoproline, homothreonine, homotryptophane, homotyrosine, homohistidine and homolysine. However, it is preferred according to the invention that an amino acid located in SEQ ID NO:1 at one of the positions 253, 305, 320, 329, 337, 338, 339 or 346 is replaced by one of the other 22 proteinogenic amino acids.

In a particularly preferred aspect of the present invention, the aspartokinase depicted in SEQ ID NO:1 has been modified by one or more of the following replacements:
(a) Thr at position 253 is modified to Ile, Leu, Gly, Phe, Trp, Tyr, Val, Pro, Cys, Met, Asp, Glu, Lys, Arg, or His;
(b) Arg at position 305 is modified to Ala, Leu, Gly, Phe, Ile, Trp, Tyr, Val, Pro, or Cys;
(c) His at position 320 is modified to Ala, Leu, Ile, Trp, Val, Pro, or Cys;
(d) Phe at position 329 is modified to Ala, Ile, Leu, Gly, Trp, Tyr, Val, Pro, Cys, Ser, Thr, Met, Asn, Gln, Asp, Glu, Lys, Arg, His;
(e) Ile at position 337 is modified to Phe, Trp, Tyr, Pro, or Cys;
(f) Ser at position 338 is modified to Ala, Ile, Leu, Gly, Phe, Trp, Tyr, Val, Pro, or Cys;
(g) Val at position 339 is modified to Ala, Ile, Leu, Gly, Phe, Trp, Tyr, Pro, Cys, Ser, Thr, Met, Asn, Gln, Asp, Glu, Lys, Arg, His;
(h) Glu at position 346 is modified to Ala, Leu, Gly, Phe, Ile, Trp, Tyr, Val, Pro, Cys, Ser, Thr, Met, Asn, Gln, Asp, Lys, Arg, His.

In a particularly preferred embodiment, the modified aspartokinase of the present invention comprises one or more of the following amino acid replacements: (a) Thr at position 253 to Arg; (b) Arg at position 305 to Ala; (c) His at position 320 to Ala; (d) Phe at position 329 to Arg; (e) Ile at position 337 to Pro; (f) Ser at position 338 to Leu; (g) Val at position 339 to Ala; (h) Glu at position 346 to Arg.

It will be appreciated that the invention is not restricted to the specific aspartokinase sequence depicted in SEQ ID NO:1. Instead, also enzymatically active homologs of the amino acid sequence of SEQ ID NO:1 may be used, which include (apart from the one or more amino acid replacements in positions 253, 305, 320, 329, 337, 338, 339 or 346) additional sequence differences when compared to the amino acid sequence depicted in SEQ ID NO:1. For example, the invention is suitable for use with aspartokinases from different species or strains of *Escherichia,* which might differ from SEQ ID NO:1 by one or more amino acid substitutions, or with enzymes isolated from a closely related genus.

Homologs of the sequence of SEQ ID NO:1 typically differ from the sequence of SEQ ID NO:1 by one or more deletions, substitutions or additions of amino acids in positions other than those corresponding to positions 253, 305, 320, 329, 337, 338, 339 or 346 of SEQ ID NO:1. Accordingly, apart from the residues which are relevant for the feedback inhibition, one or more further amino acids of the enzyme in SEQ ID NO:1 may be substituted or deleted as long as such modification does not impair the intended release from feedback inhibition, and the homolog resulting from the deletion or substitution is still an enzymatically active aspartokinase, i.e. the modification should not substantially diminish the enzyme activity of the aspartokinase. Generally, any amino acid residue of the amino acid sequences shown in SEQ ID NO:1 can be replaced by a different amino acid, provided that the resultant sequence of the variant is still an enzymatically active polypeptide with aspartokinase function. In particular, the enzyme depicted in SEQ ID NO:1 may be modified by the substitution of a total of 1, 2, 3, 4, 5, 6, 7, 8, 9, 10, 15, 20, 25, 30, 35, 40, 45 or 50 amino acids, and in some embodiments even in up 50, 60, 70, 80, 90 or 100 amino acids of the enzyme depicted in SEQ ID NO:1. Preferably, these substitutions are not relevant for the release from feedback inhibition by L-lysine.

If a polypeptide is used in the practice of the invention which includes substitutions at positions other than those disclosed herein in the context with release from feedback inhibition, i.e. in positions other than those corresponding to 253, 305, 320, 329, 337, 338, 339 or 346 of SEQ ID NO:1, it is preferred that such substitutions are conservative substitutions, i.e. substitutions of one or more amino acid residues by an amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Likewise, polypeptides which differ from the sequence depicted in SEQ ID NO: 1 by the insertion of one or more additional amino acids are considered homologs in the context of the present invention. Such insertions can be made at any position of the polypeptide shown in SEQ ID NO:1. Likewise, homologs also include polypeptides in which one or more amino acids have been deleted relative to the polypeptide shown in SEQ ID NO:1. In principle, such deletions can be applied to any amino acid position of the sequence of SEQ ID NO:1.

A homolog of the sequence of SEQ ID NO:1 shows a high degree of sequence identity with the sequence of SEQ ID NO:1. The amino acid identity will be at least about 75%, 80%, 85%, 90%, 91%, 92%, 93%, 94%, 95%, 96%, 97%, 98% or 99% when compared in an optimal alignment, for example, by the program BESTFIT using standard parameters. For example, a sequence identity of 90% means that 90% of the amino acids of an analyzed amino acid sequence stretch are identical to the sequence of the reference amino acid sequence depicted in SEQ ID NO:1. Methods and computer programs for determining amino acid sequence identity are well known in the art.

Also encompassed by the invention are enzymatically active fragments of the aspartokinase shown in SEQ ID NO:1 as well as enzymatically active fragments of the above-mentioned homologs of the aspartokinase shown in SEQ ID NO:1, provided that these fragments cover a sequence which includes one or more of the substitutions in positions corresponding to positions 253, 305, 320, 329, 337, 338, 339 or 346 in SEQ ID NO:1. Enzymatically active fragments of the sequence shown in SEQ ID NO:1 or its homologs are polypeptides that differ from the amino acid sequence shown in SEQ ID NO:1 (or from the respective homolog sequence) by the absence of one or more amino acids at the N-terminus and/or the C-terminus of the polypeptide. For example, a fragment of the sequence of SEQ ID NO:1 may differ from the sequence of SEQ ID NO:1 by the lack of about 5, 10, 15, 20, 25, 30, 35, 40, 45 or 50 amino acids at the N-terminus and/or the C-terminus, provided that such fragment retains at least a part of the enzymatic activity of the original full-length enzyme depicted in SEQ ID NO:1. Likewise, a fragment of a homolog of SEQ ID NO:1 may differ from said homolog sequence by the lack of about 5, 10, 15, 20, 25, 30, 35, 40, 45 or 50 amino acids at the N-terminus and/or the C-terminus, provided that the fragment is still enzymatically active.

The present invention also encompasses a polynucleotide encoding a polypeptide, wherein said polypeptide is an enzymatically active aspartokinase III with a reduced sensitivity to L-lysine feedback inhibition, said polypeptide comprising the amino acid sequence of SEQ ID NO:1 or an enzymatically active fragment or homolog thereof. For example, the polynucleotide of the invention can have the nucleotide sequence referred to as SEQ ID NO:1 in US Patent 5,661,012, wherein the triplets encoding amino acid positions 253, 305, 320, 329, 337, 338, 339 and 346 have been appropriately modified to provide the amino acid replacements discussed herein. Of course, the invention also encompasses polynucleotides which differ from the above-discussed polynucleotides due to the degeneracy of the genetic code.

The invention further refers to an expression vector, which comprises a polynucleotide coding for an aspartokinase enzyme of the invention with a reduced sensitivity to L-lysine feedback inhibition (or an enzymatically active fragment or homolog thereof). Generally, an expression vector refers to a polynucleotide construct, normally comprised of DNA, which allows for the genetic transfer, replication and expression of an inserted polynucleotide in a host cell, wherein said polynucleotide is not native to said host cell, or which is native to said host cell, but has been modified. Replication of the vector can be achieved either by integration of the vector into the genome of the host cell, or by episomal replication (e.g. plasmids). In the latter case, the expression vector comprises a functional origin of replication which allows for its replication in the respective host cell.

The expression vector for use in the present methods is preferably a vector having a high copy number within the host cells so as to provide for high expression levels of the aspartokinase in the host cell. The expression vector will normally comprise a gene coding for a selectable marker which is useful for identifying and selecting host cells which have been transformed with the vector. Useful markers include those which confer resistance to antibiotics, such as ampicillin, kanamycin, and tetracycline. Such markers are known in the art, and the choice of the proper marker will depend on the host cell and the vector used.

Preferred expression vectors for use in the present invention comprise a polynucleotide of the invention in a form that allows for the expression of said polynucleotide in a host cell. Normally, the expression vector will include regulatory sequences, such as promoters and enhancers, which are operatively linked to the polynucleotide selected for expression. The promoter used in the expression vector may be a constitutive or an inducible promoter. It will be appreciated that the design of the choice of the specific elements for inclusion into the expression vector will depend on the host cell to be transformed, the desired level of polypeptide expression, and the like. Preferably, the expression vectors are designed for the expression of an aspartokinase enzyme of the invention in prokaryotic cells. More preferably, the expression vectors allow for the expression of the aspartokinase of the present invention in bacterial cells, such as in cells of *E*. *coli* and C. *glutamicum.*

The expression vectors may also provide for the expression of the aspartokinase in the form of a fusion polypeptide. A fusion polypeptide refers to a non-naturally occurring hybrid polypeptide comprising at least two different polypeptides or polypeptide fragments which do occur naturally associated to each other. Expression vectors which result in the expression of fusion polypeptides normally add several amino acids to the polypeptide to be expressed, either at the N-terminus or at the C-terminus. The additional amino acid may be, for example, helpful for enhancing the expression of the aspartokinase polypeptide in the host cell, or for purifying said polypeptide after expression.

Also provided are host cells which contain a polynucleotide or an expression vector of the present invention. Different host cells can be used for the production of the aspartokinase of the invention from the expression vector. In general, both eukaryotic (e.g. yeasts or animal cells) and prokaryotic host cells can be used for recombinantly producing the aspartokinase. However, it is preferred that the host cell is a prokaryotic cell. Bacterial cells are particularly preferred. For example, the host cells may be bacteria of the genera Es*cherichia, Serratia, Brevibacterium* and *Corynebacterium,* as these bacterial hosts are frequently used in established production processes for L-lysine and L-threonine. As shown in the examples, the gene of *E*. *coli* can readily be introduced and expressed in *Corynebacterium glutamicum.* Host cells of the genus *Escherichia* are the most preferred host cells according to the present invention. Strains of *E*. *coli* that can be used for the production of L-lysine or L-threonine comprise K-12, JM109, GT3 and the like.

The host cell which is used for the amino acid production process by fermentation may be a cell which naturally possesses all biosynthetic enzymes which are required for producing L-lysine and L-threonine. These cells will be able to synthesize both amino acids. For example, a wild-type *E*. *coli* cell comprising the gene depicted in SEQ ID NO:1 may be used. However, when using the wild-type strain, the yield of L-lysine and L-threonine will be undesirably low, due to the L-lysine feedback inhibition of the aspartokinase enzyme. By transforming the cells with a polynucleotide coding for a modified aspartokinase enzyme of the invention, the yield can be significantly increased, since the modified aspartokinase enzyme has a reduced sensitivity to L-lysine feedback inhibition. Alternatively, the host cell used for the production process may be a cell which does not contain a *lysC* gene or which contains a gene coding for a non-functional aspartokinase III. In these cells, a successful transformation with a polynucleotide of the invention would be reflected by the fact that the cells start producing the amino acids of interest.

Depending on the host organism selected for transformation, a suitable expression vector will be provided. Examples for suitable vectors for polypeptide expression in *E*. *coli* cells comprise, for example, the vectors of the pBluescript series, the vectors of the pUC series, e.g. pUC18, pUC19, pBR322, pBR329, pQE70, pQE60, pQE-9, pNH8A, pNH16a, pNH18A, pNH46A, ptrc99a, pKK223-3, pKK233-3, pDR540, pRIT5, pLG338, pKC30, pHSG299, pHSG399, pRep4, pACYC177, pACYC184, pRSF1010, pBW22, and the like. Examples for expression vectors suitable for C. *glutamicum* comprise, for example, the mobilizable *E. coli*/*C. glutamicum* shuttle vectors, e.g. pEC-XT99A, pEC-XC99E, pET-XK99E. Further examples are the vectors described, e.g. in Kirchner et al., 2003, J. Biotechnol., 104:287-299, and in "Cloning Vectors" (Pouwels et al. (eds.) Elsevier, Amsterdam New York Oxford, 1985). The expression vector may be transformed into the host cell by any suitable method. The expression vector of the invention may be introduced into the host cell, e.g. by electroporation, microinjection, particle bombardement or by chemical methods such as calcium phosphate-mediated transformation, as described in Maniatis et al. 1982, Molecular Cloning, A laboratory Manual, Cold Spring Harbor Laboratory.

In a further aspect, the invention relates to a method of producing L-lysine or L-threonine, comprising the step of (i) culturing a host cell as defined above in a culture medium under conditions which allow for the expression of a polypeptide which is an enzymatically active aspartokinase III with a reduced sensitivity to L-lysine feedback inhibition, said polypeptide comprising the amino acid sequence of SEQ ID NO:1 or an enzymatically active fragment or homolog thereof, wherein one or more amino acids have been replaced at positions that correspond to amino acids 253, 305, 320, 329, 337, 338, 339 and 346 of SEQ ID NO:1, and (ii) obtaining L-lysine or L-threonine from the culture medium.

Appropriate cell culture media are well known in the art for different kinds of host cells, in particular for bacteria such as *E*. *coli.* For example, strains of *E*. *coli* can be conveniently grown in MB media, LB media and BHI media. A suitable standard minimal media for *E*. *coli* is M9. The specific media for use in culturing a host cell transformed with a polynucleotide encoding the modified aspartokinase of the invention will depend on the particular strain which is selected for the production process. The skilled person will have no problems to find appropriate media for a host cell based on the extensive literature available in the art.

Culturing of the cells expressing the aspartokinase of the invention can be performed in accordance with standard fermentation techniques. For example, the cells can be cultured in a batch or fed-batch process. Culturing methods are generally described in Encyclopedia of Bioprocess Technology - Fermentation, Biocatalysis, and Bioseparation, Volumes 1-5, Flickinger, M. C., Drew, S. W. (eds.), 1999 John Wiley & Sons. Typical temperature conditions will be in the range of 20°C to 42°C, more commonly 30°C to 40°C, and preferably 35°C to 38°C. The culture broth will typically have a pH of between 6.5 and 9.0, more typically 7.0 to 8.0, e.g. 7.5. Fermentation of the culture will be continued for a time period ranging from several hours to several days. In particular, if the culturing is performed as a batch process, the culturing time normally ranges from about 12 hours to about 36 hours. When using a continuous process, fermentation times might be up to 21 days or longer.

The purification of the products, L-threonine and L-lysine, from the culture media can be achieved by use of commonly known methods, e.g. by use of ion-exchange resins as described in U.S. Patent No. 5,342,766.

Finally, the present invention relates to the use of an aspartokinase III enzyme, which is modified as defined above, an expression vector comprising a polynucleotide encoding such aspartokinase III enzyme and/or a host cell comprising such polynucleotide and/or the expression vector for producing L-lysine or L-threonine.

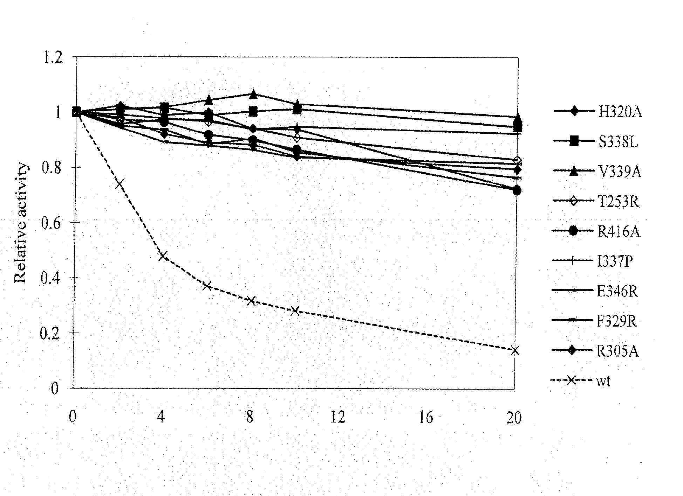

Fig. 1 shows the inhibition profiles of wild-type aspartokinase III and aspartokinase III mutants of the invention.

### EXAMPLES

### Example 1: Site-directed mutagenesis of the lysC gene

The lysc gene was amplified from chromosomal DNA of *E. coli* K12 by PCR using the following primers:
5'-GAGCCTCATATGTCTGAAATTGTTGTCTCCAA-3' (SEQ ID NO:2), and
5'-CATGCTCTCGAGCTCAAACAAATTACTATGCA-3' (SEQ ID NO:3).

The PCR reaction mixture contained:
0.5 µl *E*. *coli* K12 chromosomal DNA (0.1 mg/µl)
0.5 µl primer 1 (SEQ ID NO:2) (10 pm/µl)
0.5 µl primer 2 (SEQ ID NO:3) (10 pm/µl)
0.5 µl pfu polymerase (Fermentas Co, LTD, Germany)
5.0 µl 2 mM dNTP
5.0 µl 10 buffer (10x)
38.0 µl water

The following PCR program was used for amplification:
95°C 5 min
95°C 1 min
60°C 1 min
72°C 2 min (30 cycles)
72°C 15 min
4°C 10 min

The amplified fragment was purified and digested with the restriction enzymes NdeI and XhoI. Subsequently, the digested fragments were ligated with the pET-22b+ vector (Invitrogen GmbH, Germany) that had also been digested by *NdeI* and *XhoI*. The recombinant plasmid, designated pET-lysC, was transformed into *E. coli* JM109. The recombinant plasmid was purified and subjected to a sequence analysis to confirm the nucleotide sequence.

The plasmid pET-lysC was used as a template for subsequent mutagenesis experiments which introduced site-directed mutations into the amino acid sequence of lysc at a predetermined position. The mutagenesis experiments were carried out according to the instructions of the QuikChange II XL site-directed mutagenesis kit (Stratagene).

For the mutation of His at amino acid position 320 to Ala, the following primers were used:
5'-ACAGCCTGAATATGCTGGCTTCTCGCGGTTTCCTCG-3' (SEQ ID NO:4) and
5'-CGAGGAAACCGCGAGAAGCCAGCATATTCAGGCTGT-3' (SEQ ID NO:5).

The mutant plasmid resulting from the PCR amplification reaction using the primers of SEQ ID NO:4 and 5 was designated pET-lyse (H320A) .

For the mutation of Ser at amino acid position 338 to Leu, the following primers were used:
5'-CATCCTCGCGCGGCATAATATTTTGGTAGACTTAATCA-3' (SEQ ID NO:6) and
5'-TGATTAAGTCTACCAAAATATTATGCCGCGCGAGGATG-3' (SEQ ID NO:7).

The mutant plasmid resulting from the PCR amplification reaction using the primers of SEQ ID NO:6 and 7 was designated pET-lysC(S338L).

For the mutation of Val to Ala at amino acid position 339, a PCR was conducted by using the following oligonucleotide primers:
5'-CGCGGCATAATATTTCGGCAGACTTAATCACCACGTC-3' and (SEQ ID NO:8)
5'-GACGTGGTGATTAAGTCTGCCGAAATATTATGCCGCG-3' (SEQ ID NO:9)

The plasmid resulting from the PCR amplification reaction using the primers of SEQ ID NO:8 and 9 was pET-lysC (V339A).

For the mutation of Thr to Arg at amino acid position 253, a PCR was conducted by using the following oligonucleotide primers:
5'-CGAAGCGGCAGAGATGGCACGTTTTGGTGCAAAAGTACTG-3' (SEQ ID NO:10)
5'-CAGTACTTTTGCACCAAAACGTGCCATCTCTGCCGCTTCG-3' (SEQ ID NO:11)

The plasmid resulting from the PCR amplification reaction using the primers of SEQ ID NO:10 and 11 was pET-lysC(T253R).

For the mutation of Arg to Ala at amino acid position 305, a PCR was conducted by using the following oligonucleotide primers:
5'-CCGCGCTCTGGCGCTTGCTCGCAATCAG-3' and (SEQ ID NO:12) and
5'-CTGATTGCGAGCAAGCGCCAGAGCGCGG-3' (SEQ ID NO:13)

The plasmid resulting from the PCR amplification reaction using the primers of SEQ ID NO:12 and 13 was pET-lysC (R305A).

For the mutation of Phe to Arg at amino acid position 329, a PCR was conducted by using the following oligonucleotide primers:
5'-CGCGGTTTCCTCGCGGAAGTCGTCGGCATCCTC-3'and (SEQ ID NO:14) and
5'-GAGGATGCCGACGACTTCCGCGAGGAAACCGCG-3' (SEQ ID NO:15)

The plasmid resulting from the PCR amplification reaction using the primers of SEQ ID NO:14 and 15 was pET-lysC(F329R).

For the mutation of Ile to Pro at amino acid position 337, a PCR was conducted by using the following oligonucleotide primers:
5'-GGCATCCTCGCGCGGCATAATCCTTCGGTAGACTTAATC-3' (SEQ ID NO:16)
5'-GATTAAGTCTACCGAAGGATTATGCCGCGCGAGGATGCC-3' (SEQ ID NO:17)

The plasmid resulting from the PCR amplification reaction using the primers of SEQ ID NO:16 and 17 was pET-lysC(I337P).

For the mutation of Glu to Arg at amino acid position 346, a PCR was conducted by using the following oligonucleotide primers:
5'-GGCATCCTCGCGCGGCATAATCCTTCGGTAGACTTAATC-3' (SEQ ID NO:18)
5'-GATTAAGTCTACCGAAGGATTATGCCGCGCGAGGATGCC-3' (SEQ ID NO:19)

The plasmid resulting from the PCR amplification reaction using the primers of SEQ ID NO:18 and 19 was pET-lysC(E346R).

The sequence of all plasmids encoding a mutated amino acid sequence of aspartokinase III was confirmed by nucleotide sequencing. The plasmids were stored at -80°C or directly used for the subsequent transformation experiments.

### Example 2: Expression of mutated aspartokinase III enzymes

The plasmid pET-lysC and the plasmids encoding the mutated aspartokinase III enzymes prepared in accordance with example 1 above pET-lysC (H320A) , pET-lysC (S338L) , pET-lysC (V339A) , pET-lysC(T253R), pET-lysC(R305A), pET-lyse (I337P), pET-lyse (F329R) and pET-lyse (E34 6R) were transformed into *E*. *coli* strain BL21 (DE3) RIPL by a heat-shock method according to common protocols. The recombinant *E. coli* strains resulting from the transformation experiments were designated:
*E. coli* lysC (H320A),
*E. coli* lysC(S338L),
*E. coli* lysC (V339A),
*E. coli* lysC(T253R),
*E. coli* lysC (R305A),
*E. coli* lysC (I337PA),
*E. coli* lysC(F329R), and
*E. coli* lysC(E346R),

The recombinant *E*. *coli* strains listed above were cultured at 37°C in LB medium containing 100 mg/L ampicillin until an OD₆₀₀ of 0.6 was reached. Then, IPTG was added to the medium to a final concentration of 0.1 mM, and the bacteria were cultured at 20°C for another 12-14 h. The *E*. *coli* cells were then harvested and washed with buffer A (20 mM Tris-HC1, pH 7.5) and suspended in buffer B (20 mM Tris-HC1 (pH 7.5), 150 mM NaCl, 0.5 M ammonium sulphate). Suspended cells were disrupted by sonication and centrifuged at 13.000 rpm for 1 h. The supernatant was purified by use of a Ni²⁺-NTA column (GE Healthcare Bio-Sciences, Piscataway, NJ).

### Example 3: Measuring L-lysine feedback inhibition

The aspartokinase III activity was assayed by the method of Black and Wright by estimating the quantity of aspartate hydroxamate formed in the presence of hydroxylamine (S. Black, Methods in Enzymology, Academic Press Inc, New York, Vol. V, p. 820 (1962)). The reaction mixture contained 200 mM Tris-HC1 (pH 7.5), 10 mM MgSO₄•6H₂O, 10 mM Asp, 10 mM ATP (adjusted to pH 7.0 with KOH), 160 mM NH₂OH-HC1 (neutralized with KOH), appropriate concentrations of lysine (as an inhibitor), and the enzyme. After incubation at 30°C for 30 min, the reaction was stopped by mixing with 5% (w/v) FeCl₃ solution, and the absorbance at 540 nm was monitored.

The enzyme activities of the different aspartokinase III mutants with different lysine concentrations are shown in Fig. 1. The results show that all eight mutants (H320H, S338L, V339A, T253R, R305A, I337P, F329R, and E346R) have been released from lysine feedback inhibition even at high lysine concentrations.

### Example 4: Production of lysine in E. coli

The recombinant *E. coli* strains obtained in example 2 were used for the production of L-lysine. The fermentation medium was composed of 40 g/L glucose, 16 g/L (NH₄) ₂SO₄, 1 g/L KH₂PO₄, 1 g/L MgSO₄•7H₂O, 0.01 g/L FeSO₄•7H₂O, 0.01 g/L MnSO₄•5H₂O, 2 g/L yeast, 0.5 g/L L-methionine, 0.1 g/L L-threonine, 0.05 g/L L-isoleucine and 100 mg/L ampicillin. The recombinant *E. coli* strains were cultured at 30°C until an OD₆₀₀ of 0.6 was reached, and IPTG was added to the medium to the final concentration of 0.1 mM. The bacteria were cultured for another 30 h.

The extent of production of lysine by the different mutants is summarized in Table 1. It is shown that by the introduction of a lysine-desensitized aspartokinase III, lysine is accumulated in the medium. In contrast, the relative activity of the wild-type aspartokinase decreases with higher concentrations of lysine in the culture medium.

**Table 1: Production of lysine by mutant aspartokinase III**

| **Bacterial strain** | **Production L-lysine (g/L)** |
|---|---|
| *E. coli*/lysC (wildtype) | 0 |
| *E. coli*/lysC (H320A) | 2.12 |
| *E. coli*/lysC (S338L) | 2.34 |
| *E. coli*/lysC (V339A) | 2.55 |
| *E. coli* lysC(T253R) | 1.98 |
| *E. coli* lysC (R305A) | 2.92 |
| *E. coli* lysC (I337P) | 2.47 |
| *E. coli* lysC(F329R) | 2.55 |
| *E. coli* lysC(E346R) | 2.24 |

### Example 5: L-lysine production in Corynebacterium glutamicum

A mutated aspartokinase III gene from *E*. *coli* was also transformed into wild type C. *glutamicum* to test whether lysine production can be effected in this host. To construct a suitable expression vector for C. *glutamicum,* pET-lysC (V339A) was used as a template. A PCR was conducted by using the following oligonucleotide primers:
5'-GGCGAATTCAAAGGAGGAAAATCATGTCTGAAATTGTTGTC-3' (SEQ ID NO:20)
5'- GGCGGATCCTTACTCAAACAAATTACTATGCA-3' (SEQ ID NO:21)

The amplified fragment was purified and digested with the restriction enzymes EcoRI and *BamHI.* Subsequently, the digested fragment was ligated with the pEC-XK99E vector (Kirchner et al. (2003), J. Biotechnol. 104: 287-299) that had also been digested with *Eco*RI and *BamHI.* The resulting recombinant plasmid was designated pEC-lysC (V339A). The recombinant plasmid pEC-lysC (V339A) was subsequently transformed into wild type C. *glutamicum* ATCC 1302 by electroporation (Van der Rest et al. (1999), Appl. Microbiol. Biotechnol. 52, 541-545).

The recombinant C. *glutamicum* strain was then cultured to test whether the production of L-lysine could be effected. The fermentation medium was composed of CSL 5 g/L, MOPO 20 g/L, 50 g/L glucose, 25 g/L (NH₄)₂SO₄, 0.1 g/L KH₂PO₄, 1 g/L MgSO₄-7H₂O, 0.01 g/L FeSO₄•7H₂O, 0.005 g/L MnSO₄•5H₂O, 0.01 g/L CaCl_{2•}2H₂O, 0.3 mg/L Biotin, 0.3 mg/L Thiamine•HCl, 0.4 g/L L-homoserine, 25 g/L CaCO₃ and 100 mg/L ampicillin. The recombinant C. *glutamicum* strain was cultured at 33°C until an OD₆₀₀ of 0.6 was reached. Then, IPTG was added to the medium to the final concentration of 0.1 mM. The bacteria were cultured for another 30 h.

The production of L-lysine by the mutant and wild-type strain is summarized in Table 2. It is shown that by introduction of a lysine-desensitized aspartokinase III, lysine is accumulated in the medium. In contrast, the wildtype C. *glutamicum* does not produce any lysine.

**Table 2: Production of lysine in C. glutamicum**

| **Bacterial strain** | **Production L-lysine (g/L)** |
|---|---|
| C. *glutamicum* (wildtype) | 0 |
| C. *glutamicum*/lysC (V339A) | 2.75 |

## Claims

1. Polypeptide which is an enzymatically active aspartokinase III with a reduced sensitivity to L-lysine feedback inhibition, said polypeptide comprising the amino acid sequence of SEQ ID NO:1 or an enzymatically active fragment or homolog thereof, wherein one or more amino acids have been replaced at positions that correspond to amino acids 253, 305, 320, 329, 337, 338, 339 and 346 of SEQ ID NO:1.

2. Polypeptide of claim 1, wherein the amino acid replacements are one or more of the following replacements:
(a) Thr 253 to Ile, Leu, Gly, Phe, Trp, Tyr, Val, Pro, Cys, Met, Asp, Glu, Lys, Arg, or His;
(b) Arg 305 to Ala, Leu, Gly, Phe, Ile, Trp, Tyr, Val, Pro, or Cys;
(c) His 320 to Ala, Leu, Ile, Trp, Val, Pro, or Cys;
(d) Phe 329 to Ala, Ile, Leu, Gly, Trp, Tyr, Val, Pro, Cys, Ser, Thr, Met, Asn, Gln, Asp, Glu, Lys, Arg, His
(e) Ile 337 to Phe, Trp, Tyr, Pro, or Cys;
(f) Ser 338 to Ala, Ile, Leu, Gly, Phe, Trp, Tyr, Val, Pro, or Cys;
(g) Val 339 to Ala, Ile, Leu, Gly, Phe, Trp, Tyr, Pro, Cys, Ser, Thr, Met, Asn, Gln, Asp, Glu, Lys, Arg, His
(h) Glu 346 to Ala, Ile, Leu, Gly, Phe, Trp, Tyr, Val, Pro, Cys, Ser, Thr, Met, Asn, Gln, Asp, Lys, Arg, His

3. Polypeptide of claim 1 or 2, wherein the amino acid replacements are one or more of the following replacements:
(a) Thr 253 to Arg;
(b) Arg 305 to Ala;
(c) His 320 to Ala;
(d) Phe 329 to Arg;
(e) Ile 337 to Pro;
(f) Ser 338 to Leu;
(g) Val 339 to Ala;
(h) Glu 346 to Arg;

4. Polypeptide of claim 1-3, wherein the sensitivity to L-lysine feedback inhibition is reduced at least 2-fold, at least 5-fold, or at least 10-fold.

5. Polypeptide of claim 1-4, wherein said polypeptide is substantially free from L-lysine feedback inhibition.

6. Polynucleotide encoding a polypeptide of claims 1-5.

7. Expression vector comprising the polynucleotide of claim 6.

8. Host cell comprising the polynucleotide of claim 6 or the expression vector of claim 7.

9. Host cell of claim 8, wherein the host cell is a prokaryotic cell.

10. Host cell of claim 9, wherein the host cell is a bacterial cell.

11. Host cell of claim 10, wherein the bacterial cell is an *Escherichia* cell.

12. Host cell of claim 10, wherein the bacterial cell is a *Corynebacterium* cell.

13. Method of producing L-lysine or L-threonine, comprising the steps of (i) culturing a host cell of any of claims 8-10, which is capable of producing L-lysine, in a culture medium under conditions which allow for the expression of a polypeptide of any of claims 1 to 5, and (ii) obtaining L-lysine or L-threonine from the culture medium.

14. Use of a polypeptide of any of claims 1 to 5, an expression vector of claim 7 or a host cell of claims 8-12, for producing L-lysine or L-threonine.
